Europäisches Patentamt

European Patent Office

Office européen des brevets

(11) Publication number: **0 348 190**
A1

# EUROPEAN PATENT APPLICATION

(21) Application number: **89306288.5**

(22) Date of filing: **22.06.89**

(51) Int. Cl.4: **C 07 C 19/08**
C 07 C 17/20, C 07 C 17/00

(30) Priority: **23.06.88 US 210553**

(43) Date of publication of application:
**27.12.89 Bulletin 89/52**

(84) Designated Contracting States:
**AT BE CH DE ES FR GB GR IT LI LU NL SE**

(71) Applicant: **E.I. DU PONT DE NEMOURS AND COMPANY**
**(a Delaware corporation)**
**1007 Market Street**
**Wilmington Delaware 19898 (US)**

(72) Inventor: **Rao, Velliyur Nott Mallikarjuna**
**1, Georgetown Avenue**
**Wilmington Delaware 19809 (US)**

(74) Representative: **Jones, Alan John et al**
**CARPMAELS & RANSFORD 43 Bloomsbury Square**
**London, WC1A 2RA (GB)**

(54) Catalyzed hydrofluorination process.

(57) A process for preparing fluorinated alkanes by contacting alkenes or halogenated alkanes with from 1 to 30 molar equivalents of hydrogen fluoride at from about 0°C to about 185°C in the presence of from 0.001 to 0.250 molar equivalent of $NbCl_5$ or $NbBr_5$.

**Description**

## Catalyzed Hydrofluorination Process

### FIELD OF THE INVENTION

Process for the preparation of fluorinated alkanes by contacting alkenes or halogenated alkanes with hydrogen fluoride in the presence of niobium pentachloride ($NbCl_5$) or niobium pentabromide ($NbBr_5$).

### BACKGROUND OF THE INVENTION

A. E Feiring, Journal of Fluorine Chemistry, 13, 7-18 (1979) discloses the use of tantalum pentafluoride and niobium pentafluoride as a catalyst for the addition of hydrogen fluoride to tetra- and trichlorethane and related compounds. The catalyst is also useful for fluorine chlorine exchange reactions. However, under the conditions of the batch experiments [$HF/CCl_2 = CCl_2 = 2.5$, temp $= 150°C$, reaction time = six hours] catalysts such as $BF_3$, $TaCl_5$, $Ta_2O_3$, $CoF_3$, $V_2O_5$, $ZrCl_4$, $NbCl_5$, $HgO$, and $WC16$ showed no catalytic activity for the addition of HF to tetrachloroethylene.

The use of tantalum pentafluoride and niobium pentafluoride as a catalyst for the addition of hydrogen fluoride to unsaturated compounds has been disclosed and claimed in U.S. 4,258,225.

U.S. 4,374,289 discloses the addition of HF to $CHCl = CCl_2$ using $NbCl_5$ as a catalyst to produce $CH_2ClCCl_2F$. Conditions for the reaction are $HF/CHCl = CCl_2 = 1.5$ to 3.5, temp. $50°C$ to $150°C$, reaction time = 15 minutes to 5 hours, $NbCl_5 = 0.05$ to 0.2 mole/mole $CHCl = CCl_2$ and pressure = 175 to 250 psig. The reaction is conducted under moderate temperature conditions in order to avoid overfluorination.

This invention provides conditions under which $NbCl_5$ or $NbBr_5$ functions as a mild hydrofluorination catalyst and is capable of fluorinating even less reactive molecules such as $CCl_2 = CCl_2$.

### SUMMARY OF THE INVENTION

This invention is a process for the preparation of fluorinated alkanes by contacting at a temperature from about $0°C$ to about $185°C$ under substantially anhydrous conditions, one molar equivalent of a starting material selected from alkenes or halogenated alkanes of the following formulas
$R^1R^2C = CR^3R^4$ and $R^5R^6R^7R^8C$
wherein $R^1$, $R^2$, $R^3$, $R^4$, $R^5$, $R^6$, $R^7$ are H, F, Cl, Br, or $CZ_3$, wherein Z is selected from at least one of the group consisting of H, F, Cl, or Br and wherein $R^8$ is Br or Cl, with the proviso that when $R^1$ is H only two of $R^2$, $R^3$ and $R^4$ can be Cl, with HF, the mole ratio of HF to alkene being from 4 to 30 and the mole ratio of HF to halogenated alkane being from 1 to 30, in the presence of 0.001 to 0.250 molar equivalent of at least one catalyst selected from $NbCl_5$ and $NbBr_5$.

### DETAILS OF THE INVENTION

The resulting fluorinated alkane produced in accordance with this invention has one or more fluorine atoms over and above the number of fluorine atoms originally present in the alkene or halogenated alkane used as the starting material.

The alkene or halogenated alkane starting materials of the invention do not substantially react with hydrogen fluoride alone under the conditions of temperature and pressure used in this invention and require the presence of added catalyst specifically $NbCl_5$ or $NbBr_5$. Preferred alkene of the formula $R^1R^2C = CR^3R^4$ is wherein $R^1$, $R^2$, $R^3$, and $R^4$ are Cl, i.e. tetrachloroethylene, $Cl_2C = CCl_2$.

The preferred halogenated alkanes of the formula $R^5R^6R^7R^8$ are wherein $R^7$ is $CHCl_2$, $CH_2Cl$, $CH_3$, Cl, or H, when $R^5$, $R^6$, and $R^8$ are Cl; and wherein $R^5$ and $R^6$ are H when $R^7$ and $R^8$ are Cl. The specifically preferred halogenated alkanes are $CCl_3CHCl_2$, $CCl_3CH_2Cl$, $CCl_3CH_3$, $CCl_4$, $CHCl_3$, and $CH_2Cl_2$.

The reaction can be carried out in the liquid phase or vapor phase and at autogenous pressures or under constant pressure ranging from atmospheric to superatmospheric. Both the liquid phase and vapor phase processes include batch, semicontinuous and continuous modes of operation.

It is preferred that the $NbCl_5$ or $NbBr_5$ be used in an amount from 0.001 to 0.250 moles per mole of starting alkene or halogenated alkane for reasons of economy and effectiveness. The catalyst is a commercially available crystalline solid and can be used alone or on a support such as carbon.

The reaction can be carried out at from about $0°C$ to about $185°C$. The preferred temperature is from about $35°C$ to about $165°C$.

Anhydrous or substantially anhydrous conditions means that water, which is detrimental to the reaction, should be excluded as much as possible from the reaction zone. The HF which is commercially available as anhydrous grade can be used in the reaction directly. Exclusion of moisture from the reaction vessel by means of appropriate moisture traps or other means is a routine procedure and is well known in the art.

It is preferred that 1 to 30 molar equivalents of HF, relative to the halogenated alkane, be utilized for reaction with halogenated alkanes and that 4 to 30 molar equivalents of HF, relative to the alkene, be utilized for reaction with alkenes. At least 5 molar equivalents of HF is preferred, particularly for highly chlorinated alkenes such as $CCl_2 = CCl_2$. In practice from 15 to 30 molar equivalents of HF ensures the best combination of economics and fluorination yield.

The reaction vessel is constructed from materials which are resistant to the action of hydrogen halide such as monel, "Hastelloy" and "Inconel".

The liquid phase reactions are conducted by introducing the reagents in any order into the reaction vessel. Generally, the NbCl₅ or NbBr₅ and starting alkene or halogenated alkane are placed in the reaction vessel which is then cooled, and the required amount of hydrogen fluoride is condensed in the vessel. The vessel may be evacuated prior to the introduction of hydrogen fluoride and cooled in Dry Ice or liquid nitrogen to facilitate addition of the hydrogen fluoride. The contents of the vessel are raised to the appropriate reaction temperature and agitated by shaking or stirring for a length of time sufficient to cause the reaction to occur.

For liquid phase reactions, the amount of NbCl₅ or NbBr₅ used is from 0.001 to 0.250 mole per mole of starting alkane or halogenated alkane, and is more preferably from 0.005 to 0.1 moles per mole of starting alkene or halogenated alkane. The amount of HF used in the reaction is from 1 to 30 molar equivalents per mole of halogenated alkane and from 4 to 30 molar equivalents per mole of alkene. The reaction can be carried out at from about 0°C to 185°C. The preferred temperature is about 35°C to about 165°C. The reaction time can be from 0.5 to 18 hours; the preferred times are from 1 to 8 hours.

In the vapor phase reaction, the reactants are introduced into the reactor above their boiling points. The temperature of the reactor must also be sufficient to keep the products of the reaction in the vapor state so that they pass over into a cooled receiver beyond the reactor rather than remain in the catalyst zone for a prolonged period of time.

For vapor phase reactions, it is convenient to support the NbCl₅ or NbBr₅ on an inert porous material such as carbon or other known supports. The amount of catalyst to inert support is from 10% to 50% with amounts of about 25% being preferred. The amount of HF used in the reaction is from 1 to 30 molar equivalents per mole of halogenated alkane and from 4 to 30 molar equivalent per mole of alkene. The reaction can be carried out at from about 50°C to about 185°C. The preferred temperature is about 70°C to about 170°C. The contact time of the reagents with the catalyst may be specified instead of reaction time. The combined operations of feed rate, control of reactor temperature and pressure and rate of removal of product from the reactor influence the residence time of the product in the reactor. It may be desirable to shorten the residence time for a given product within the reactor to control the formation of undesired products. Contact time is the average time that the reactant product mixture is in contact with the catalyst. Broadly, contact times of from 0.1 to 25 seconds are useful with preferred contact times in the range of 1 to 10 seconds.

Under the reaction conditions set forth above a portion of the NbCl₅ or NbBr₅ may be in the form of NbCl₅₋ₓFₓ or NbBr₅₋ₓFₓ. The instant invention is understood to include the condition where it may exist.

Pressure is not critical. Atmospheric, superatmospheric and autogeneous pressures are the most convenient and are therefore preferred.

The fluorinated alkanes produced by the invention have utility as refrigerants, solvents and blowing agents.

## EXAMPLE

General Experimental Procedure

The reactor consisted of a 100 ml high pressure cylinder made of monel or "Inconel" containing a magnetic stirrer and an internal thermocouple. Mounted on top of the reactor was a condenser and a back pressure regulator connected to an optional on line analytical system. Suitable inlet and exit lines were also present to allow for admission of reactants and withdrawal of products.

To the reactor was charged NbCl₅ in the desired amount. The reactor was then cooled and evacuated. The alkene or halogenated alkane starting material and the required amount of HF were then admitted to the reactor. It was then pressurized with nitrogen to the desired pressure while still cold and then gradually brought to the desired operating temperature with stirring by using external heat provided by an oil bath. The back pressure regulator was set to the desired operating pressure prior to heating the reactor.

At the completion of the reaction, the product was isolated by conventional means and analyzed by gas chromatography. All of the percentages reported in the Examples are area %.

## EXAMPLE 1

The General Experimental Procedure was followed using 16.5 g of CCl₂=CCl₂, 3.0 of NbCl₅ and 15 g of anhydrous HF [Molar ratio of HF/CCl₂=CCl₂ = 7.5/1]. The reactor was pressurized to 200 psig when cold with nitrogen and the back pressure regulator was set for 500 psig. The contents were heated with stirring at 142-148°C for about 2 hours. Product analysis indicated 85.0% CClF₂CHCl₂ and 10.9% CF₃CHCl₂, in addition to small amounts of other organics.

## EXAMPLE 2

The General Experimental Procedure was followed using 20.25 g of CHCl₂CCl₃, 3.0 g of NbCl₅ and 15 g of anhydrous HF [Molar ratio of HF/CHCl₂CCl₂ = 7.5/1]. The reactor was pressurized to 200 psig when cold with nitrogen and the back pressure regulator was set for 500 psig. The contents were heated with stirring at 142-148°C for three hours. Product analysis indicated 92.5% CClF₂CHCl₂ and 5.1% CF₃CHCl₂, and small amounts of other organics.

## EXAMPLE 3

The General Experimental Procedure was followed using 30.8 g of CCl₄, 3.0 g of NbCl₅ and 10 g of anhydrous HF [Molar ratio of HF/CCl₄ = 2.5/1]. The reactor was pressurized to 200 psig when cold with nitrogen and the back pressure regulator was set for 400 psig. The contents were heated with stirring at 80-85°C for about 45 minutes. An off gas analysis during this period showed greater than 98% $CF_2Cl_2$. The pressure was carefully vented to remove most of the $CF_2Cl_2$. The product remaining in the reactor (24.0g) was analyzed and found to contain 1.3% $CF_2Cl_2$, 21.2% $CFCl_3$ and 77.5% of $CCl_4$.

## EXAMPLE 4

The General Experimental Procedure was followed using 34.0 g $CH_3CCl_3$, 0.3 g $NbCl_5$ and 5.0 g anhydrous HF [Molar ratio of $HF/CH_3CCl_3$ = 1/1]. The reactor was pressurized to 100 psig when cold with nitrogen and the back pressure regulator was set for 200 psig. The contents were heated with stirring at 36=37°C for about 30 minutes. Product analysis showed 7.6% $CH_3CClF_2$, 46.3% $CH_3CCl_2F$ and 43.5% $CH_3CCl_3$ in addition to small amounts of other organics.

## EXAMPLE 5

Example 4 was substantially repeated except that 0.5 g $NbCl_5$ was used. Product analysis indicated 12.1% $CH_3CClF_2$, 57.3% $CH_3CCl_2F$, and 28.2% $CH_3CCl_3$ in addition to small amounts of other organics.

**Claims**

1. A process for the preparation of fluorinated alkanes by contacting at a temperature from about 0°C to about 185°C under substantially anhydrous conditions, one molar equivalent of a starting material selected from alkenes or halogenated alkanes of the following formulas
$R^1R^2C = CR^3R^4$ and $R^5R^6R^7R^8C$
wherein $R^1$, $R^2$, $R^3$, $R^4$, $R^5$, $R^6$, $R^7$ are H, F, Cl, Br, or $CZ_3$, wherein Z is selected from at least one of H, F, Cl, or Br, and
wherein $R^8$ is Br or Cl, with the proviso that when $R^1$ is H, only two of $R^2$, $R^3$ and $R^4$ can be Cl.
with HF, the mole ratio of HF to alkene being from 4 to 30 and the mole ratio of HF to halogenated alkane being from 1 to 30,
in the presence of 0.001 to 0.250 molar equivalent of at least one catalyst selected from $NbCl_5$ and $NbBr_5$.

2. The process of Claim 1 wherein starting material is $Cl_2C = CCl_2$.

3. The process of Claim 1 wherein the starting material is selected from $CCl_3CHCl_2$, $CCl_3CH_2Cl$, $CCl_3CH_3$, $CCl_4$, $CHCl_3$ and $CH_2Cl_2$.

4. The process of any one of Claims 1 to 3 wherein the catalyst is $NbCl_5$.

5. The process of any one of Claims 1 to 4 wherein the temperature is from about 135°C to about 165°C.

6. The process of any one of Claims 1 to 5 wherein the mole ratio of HF to starting material is from 15 to 30.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-4 258 225  (FEIRING)  --- | | C 07 C  19/08 |
| A | US-A-2 005 707  (H.W. DAUDT et al.)  * Claim 3 *  --- | | C 07 C  17/20 |
| D,X | US-A-4 374 289  (VAN DER PUY et al.)  * Abstract *  ----- | 1,4,5 | C 07 C  17/00 |

TECHNICAL FIELDS
SEARCHED (Int. Cl.4)

C 07 C   17/00

The present search report has been drawn up for all claims

| Place of search | Date of completion of the search | Examiner |
|---|---|---|
| THE HAGUE | 13-09-1989 | VAN GEYT J.J.A. |